# EUROPÄISCHE PATENTSCHRIFT

(11) **EP 1 980 365 B1**
(45) Veröffentlichungstag und Bekanntmachung des Hinweises auf die Patenterteilung: **29.12.2010**
(21) Anmeldenummer: 08101733.7
(22) Anmeldetag: 05.04.2007
(51) Int. Cl.: B24C 1/00

(54) **Vorrichtung und Verfahren zur Oberflächenbearbeitung bzw. Oberflächenbehandlung mittels Trockeneisgranulat**
Device and method for processing surfaces or surface processing using dry ice granules
Dispositif et procédé destinés au traitement de surface ou à la manipulation de la surface au moyen d'un granulé de glace sèche

(43) Veröffentlichungstag der Anmeldung: 15.10.2008
(62) Teilanmeldung aus: 07105772.3
(73) Patentinhaber: Rotstein, Rosa, 80797 München (DE)
(72) Erfinder: Rotstein, Rosa, 80797 München (DE)
(74) Vertreter: Farago, Peter Andreas

(56) Entgegenhaltungen:
- EP-A- 0 234 365
- WO-A-90/14927
- WO-A-2004/037098
- US-A1- 2002 068 511
- US-A1- 2006 178 092
- US-B1- 6 174 225

## Beschreibung

Die vorliegende Erfindung betrifft eine Vorrichtung und ein Verfahren zur Oberflächenbearbeitung bzw. Oberflächenbehandlung mittels Trockeneisgranulat.

### Stand der Technik

Trockeneis bzw. Trockeneisgranulat besteht, wie dem Fachmann wohl bekannt, aus einem Kohlendioxid (handelsüblich auch Kohlensäure genannt) in festem Zustand, das üblicherweise in Form von Pellets in einer Größe von etwa 3 mm erhältlich ist.

Aus der DE 10 2004 045 770 B3 ist, wie in Fig. 1 schematisch dargestellt, eine Vorrichtung zum bestrahlen einer Fläche mit einem Gemisch aus Treibgas (Transportluft) und Trockeneisgranulat bekannt, die nach dem Druckstrahlprinzip bzw. Einschlauchprinzip arbeitet.

Die bekannte Vorrichtung umfasst einen Einlass 6 für die Treibgasversorgung, der über einen durch eine Rotationskammer 90 verlaufenden Strömungsweg mit einem Auslass 22 in Verbindung steht, an den ein Transportschlauch 8 zum Ausgeben des Gemisches aus Treibgas und Trockeneisgranulat über eine Strahlpistole 9 mit einer Lavaldüse 10 anschließbar ist.

Darüber hinaus umfasst die bekannte Vorrichtung eine Dosiereinrichtung 20 zum Einbringen des Trockeneisgranulats in den Strömungsweg des Treibgases, wobei die Dosiereinrichtung 20 eine motorisch um eine Mittelachse drehbare Dosierscheibe 30 umfasst, die zwischen einem plattenförmigen Einströmteil 32 und einem plattenförmigen Ausströmteil 34 angeordnet ist und eine Vielzahl von Aufnahmekammern 44 aufweist, die in einer ersten Drehstellung der Dosierscheibe 30 fluchtend zu einer Zufuhrkammer 57 des Ausströmteiles 34 und in einer zweiten Drehstellung der Dosierscheibe 30 zwischen einer Einströmkammer 71 des Einströmteiles 32 und einer Ausströmkammer 59 des Ausströmteiles 34 um eine Antriebsachse 41 positionierbar sind. Zur Drehung der Dosierscheibe 30 wird ein herkömmlicher Motor 36 mit einer Drehzahlregelung 39 vorgesehen.

Die Zufuhrkammer 57 ist mit einem trichterförmigen Vorratsbehälter 1 zum Einbringen von Trockeneisgranulat in die Zufuhrkammer 57 verbunden, wobei der Vorratsbehälter mit herkömmlichen Trockeneis-Pellets 2 ausgefüllt ist, die unter Einwirkung der Schwerkraft und mit Unterstützung eines Rüttlers 86 in die Zufuhrkammer 57 gelangen können. Von der Zufuhrkammer 57 gelangen die Pellets 2 unter Einwirkung der Schwerkraft in eine der Aufnahmekammern 44 in der ersten Drehstellung und werden sodann durch die Drehung der Dosierscheibe 30 in die zweite Drehstellung befördert, so dass sie in die Durchströmungsrichtung 93 in Richtung des Auslasses 22 und zur Strahlpistole 9 hin befördert werden, um anschließend druckbeaufschlagt auf die zu reinigende bzw. zu behandelnde Fläche 28 aufzutreffen.

Die Vorrichtung der DE 10 2004 045 770 B3 ist jedoch insofern nachteilig, da ihre Reinigungsleistung relativ begrenzt ist und der Trockeneisverbrauch relativ hoch ist. Eine Erhöhung der Reinigungsleistung der Vorrichtung gemäß DE 10 2004 045 770 B3 wäre zwar durch die Steigerung der Menge an Treibgas möglich, was allerdings eine entsprechende Ausgestaltung und Dimensionierung der Druckluftquelle mit nachteiligen Auswirkungen für die Handhabbarkeit der Vorrichtung erfordern würde. Darüber hinaus ist die Vorrichtung gemäß der DE 10 2004 045 770 B3 nicht für topische und schonende Anwendungen wie z. B. die kosmetische Behandlung bzw. Reinigung der Haut nicht geeignet.

Dokument WO 90 14927 zeigt in Abbildung 4 eine Vorrichtung zur Oberflächenbearbeitung bzw. Oberflächenbehandlung mittels Eisgranulat mit einem Vorratsbehälter zur Aufnahme von Eis-Pellets, einem Einlass für die Zuführung von Druckluft an einen Strömungskanal, einem Auslass zur Zuleitung eines Gemisches aus Druckluft und Eisgranulat an eine Strahlpistole und einer mit dazwischenliegenden Ausströmkammer, die mit dem Strömungskanal verbunden ist,
wobei im Pfad zwischen dem Vorratsbehälter und der Ausströmkammer ein radiales Mahlwerk angeordnet ist, das ausgebildet ist, um die Eis-Pellets zu einer Größe in einem vorgegebenen Bereich zu zerkleinern,
das Mahlwerk eine Brechwalze umfasst, die ausgebildet ist, um die Form der zerkleinerten Eis-Pellets scharfkantig zu gestalten;
das Mahlwerk ausgebildet ist die Größe der zerkleinerten Eis-Pellets in einem Bereich von etwa 1 mm bis etwa 100 µ variabel einzustellen;
weiterhin eine radiale Transportwalze umfasst, die zwischen dem Mahlwerk und der Ausströmkammer angeordnet ist, wobei die Transportwalze mit einer veränderlichen Drehzahl betreibbar ist, um die Menge der zerkleinerten Eis-Pellets pro Zeiteinheit zu variieren.

Der Erfindung liegt die Aufgabe zugrunde, eine Vorrichtung und ein Verfahren zur Oberflächenbearbeitung bzw. Oberflächenbehandlung mittels Tockeneisgranulat zu schaffen, die bzw. das die oben angeführten Probleme des Standes der Technik vermeidet.

Im Rahmen dieser Aufgabe besteht eine besondere Aufgabe der vorliegenden Erfindung darin, eine Vorrichtung und ein Verfahren zur Oberflächenbearbeitung bzw. Oberflächenbehandlung mittels Trockeneisgranulat zur Verfügung zu stellen, die bzw. das Reinigungsleistung (Aggressivität) erhöht und gleichzeitig den Verbrauch an Trockeneisgranulat und/oder die Menge des Treibgases (Transportluft) senkt.

Eine weitere Aufgabe der Erfindung besteht darin, eine Vorrichtung und ein Verfahren zur Oberflächenbearbeitung bzw. Oberflächenbehandlung mittels Trockeneisgranulat zu schaffen, die bzw. das für topische und schonende Anwendungen wie z. B. die kosmetische Behandlung bzw. Reinigung der Haut geeignet ist.

Desweiteren besteht eine besondere Aufgabe der vorliegenden Erfindung darin, eine Vorrichtung und ein Verfahren zur Oberflächenbearbeitung bzw. Oberflächenbehandlung mittels Trockeneisgranulat zu realisieren, die bzw. das leicht und kostengünstig herstellbar ist.

Die obigen Aufgaben und weitere der nachfolgenden Beschreibung zu entnehmende Aufgaben werden durch eine Vorrichtung und ein Verfahren gemäß den anliegenden Hauptansprüchen gelöst. Vorteilhafte Weiterbildungen der gegenwärtigen Erfindung sind Gegenstand der Unteransprüche.

### Kurzbeschreibung der Figuren

Weitere Merkmale und Vorteile der vorliegenden Erfindung sowie die Wirkungsweise der exemplarischen Ausführungsform der vorliegenden Erfindung werden unten mit Bezug auf die begleitenden Zeichnungen beschrieben. Die begleitenden Zeichnungen veranschaulichen die vorliegende Erfindung und dienen zusammen mit der Beschreibung weiterhin dazu, die Grundsätze der Erfindung zu erklären und es einem Fachmann auf dem betreffenden Gebiet zu ermöglichen, die Erfindung herzustellen und zu verwenden.

Dabei zeigen:
Fig. 1 eine schematische Darstellung einer bekannten Vorrichtung gemäß der DE 10 2004 045 770 B3;
Fig. 2 eine schematische Darstellung einer ersten Ausführungsform der erfindungsgemäßen Vorrichtung zur Oberflächenbearbeitung bzw. Oberflächenbehandlung mittels Trockeneisgranulat; und
Fig. 3 eine schematische Darstellung einer zweiten Ausführungsform der erfindungsgemäßen Vorrichtung zur Oberflächenbearbeitung bzw. Oberflächenbehandlung mittels Trockeneisgranulat.

### Beschreibung der bevorzugten Ausführungsformen der Erfindung

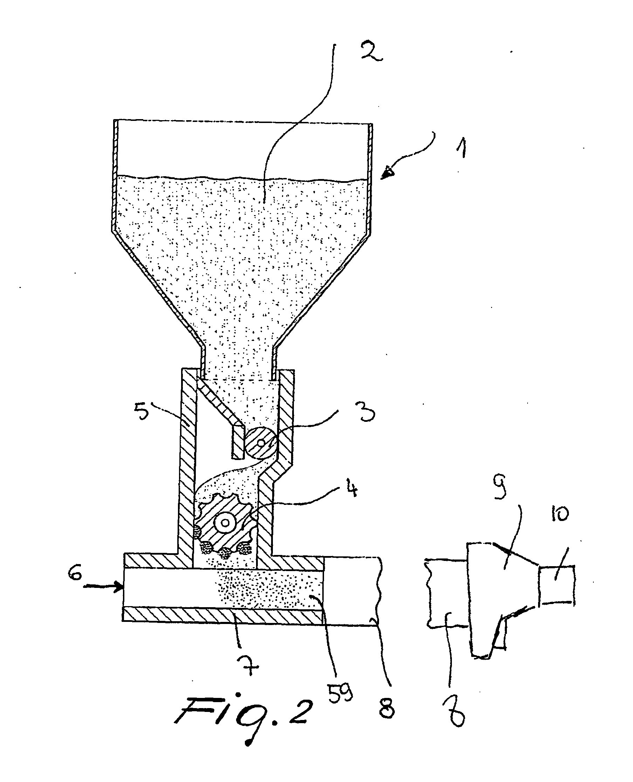

Unter Bezugnahme auf Fig. 2 wird eine schematische Darstellung einer ersten Ausführungsform der erfindungsgemäßen Vorrichtung zur

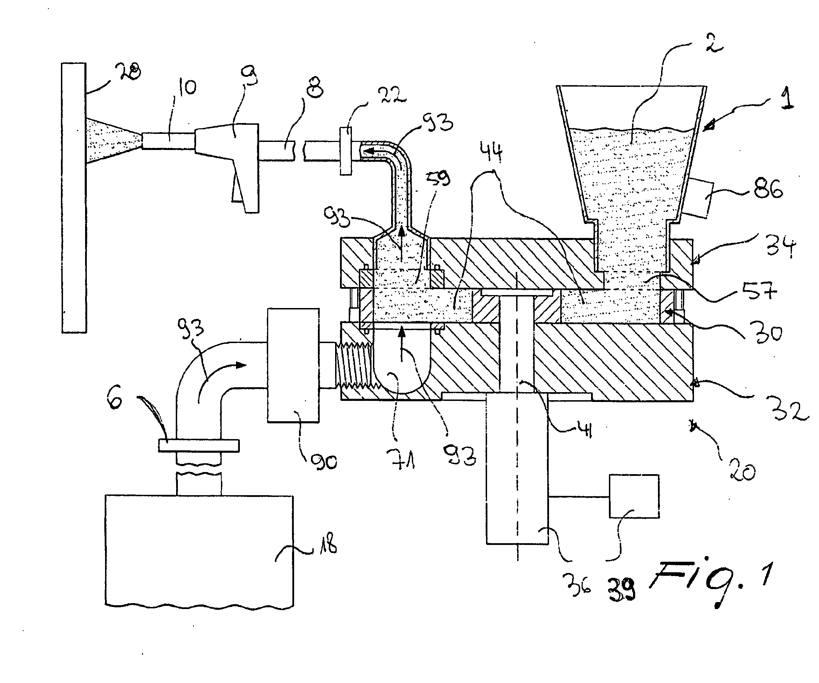

Oberflächenbearbeitung bzw. Oberflächenbehandlung mittels Trockeneisgranulat erläutert, wobei gemeinsame Bestandteile zur Fig. 1 mit gleichen Bezugszeichen versehen werden. Die erfindungsgemäße Vorrichtung umfasst einen im Wesentlichen trichterförmigen Vorratsbehälter 1 der mit Trockeneis-Pellets 2 (nachstehend zur Vereinfachung auch Pellets genannt) aufgefüllt ist. Die Pellets 2 haben, wie handelsüblich erhältlich, eine Größe von etwa 3 mm.

Erfindungsgemäß mündet der Vorratsbehälter 2 in ein radial ausgebildetes einstellbares Mahlwerk 3, welches die durch Schwerkraft aufliegenden 3 mm Pellets 2 in kleine Partikel zerkleinert, die in einem Bereich von etwa 1 mm bis etwa 100 µ liegen. Besonders bevorzugt ist ein Zerkleinerungsbereich der Pellets 2 in einer Größenordnung von etwa 200 µ bis etwa 400 µ und besonders bevorzugt sind Partikel, die etwa 300 µ groß sind.

Vorteilhaft ist das radiale Mahlwerk 3 ausgebildet, um scharfkantige zerkleinerte Partikel zu erzeugen. Die Scharfkantigkeit der zerkleinerten Partikel, die sich unterstützend auf die Reinigungsleistung auswirkt, kann durch eine angepasste Oberflächengeometrie der Brechwalze des Mahlwerks 3 erzielt werden.

Das radiale Mahlwerk 3 wird von einem nicht gezeigten Motor drehend angetrieben, wobei vorteilhaft die Menge der zerkleinerten Partikel pro Zeiteinheit durch die Regelung der Drehzahl des Motors bzw. der Drehzahl der Brechwalze des Mahlwerks 3 variierbar ist.

Die zerkleinerten Partikel aus dem Mahlwerk 3 treffen unter Einwirkung der Schwerkraft auf eine radial ausgebildete Transportwalze 4, die mit axial verlaufenden Einkerbungen 4' versehen ist. Vorzugsweise erstrecken sich die Einkerbungen 4' über die gesamte axiale Länge der Transportwalze 4. Darüber hinaus ist es erfindungsgemäß denkbar, senkrecht zu den axialen Einkerbungen 4' radial verlaufende (nicht gezeigte) Einkerbungen zu realisieren. Die Transportwalze 4 ist vorzugsweise ebenfalls motorisiert, um ihre Drehzahl zu regeln, damit die Menge der zerkleinerten Partikel pro Zeiteinheit variiert wird.

Wie in Fig. 2 gezeigt, werden die in die Einkerbungen aufgenommenen zerkleinerten Partikel zwischen der Transportwalze 4 und der senkrechten Wand 5 der Zufuhrkammer 57 gezwungen. Dementsprechend ist es erfindungsgemäß denkbar, die Form bzw. Scharfkantigkeit der zerkleinerten Partikel durch den Abstand zwischen der senkrechten Wand 5 und der Transportwalze 5 zu beeinflussen. Dies kann durch eine (nicht gezeigte) tulpen- bzw. trichterfömige Ausgestaltung der Wand 5 und durch eine relative senkrechte Verschiebung der Wand 5 und/oder der Transportwalze 4 bewerkstelligt werden, um somit den Abstand dazwischen zu variieren.

Durch die Rotationsbewegung der Transportwalze 4 fallen die zerkleinerten Partikel in einen um 90° versetzten Strömungskanal 7 der erfindungsgemäßen Vorrichtung, der in einer herkömmlichen Weise, wie z. B. in der DE 10 2004 045 770 B3 beschrieben, am Einlass 6 von einer Druckluftquelle 18 mit Druckluft beaufschlagt wird. Durch die Einwirkung von Druckluft gelangen die zerkleinerten Partikel in den Transportschlauch 8 und von dort aus in die Strahlpistole 9 bzw. die Lavaldüse 10 der Strahlpistole 9.

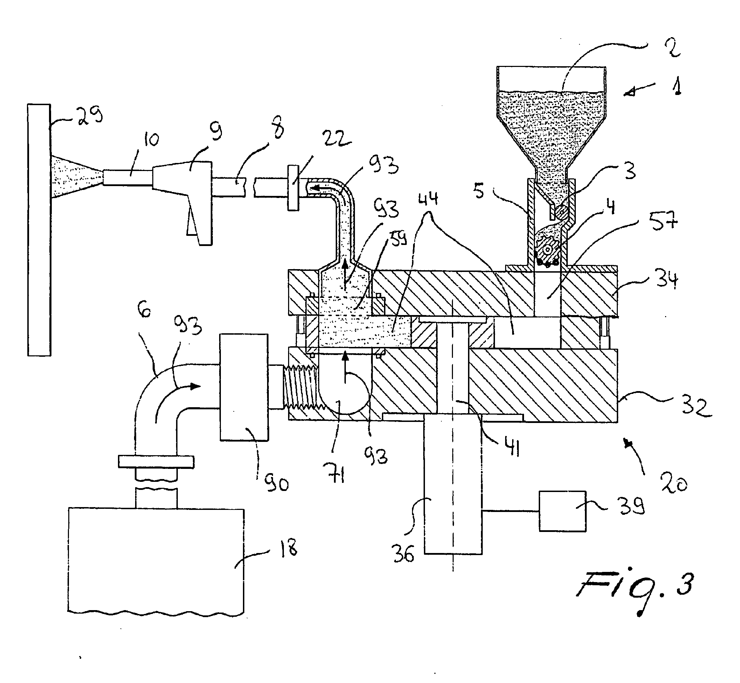

Unter Bezugnahme auf Fig. 3 wird eine schematische Darstellung einer zweiten Ausführungsform der erfindungsgemäßen Vorrichtung zur Oberflächenbearbeitung bzw. Oberflächenbehandlung mittels Trockeneisgranulat erläutert, wobei gemeinsame Bestandteile zu den Figuren 1 und 2 mit gleichen Bezugszeichen versehen werden.

Die zweite Ausführungsform setzt den Vorratsbehälter 1, das Mahlwerk 3 und die Transportwalze 4 der ersten Ausführungsform in einer Vorrichtung 1 ein, die in Verbindung mit einer Dosiereinrichtung 20 der DE 10 2004 045 770 B3 zum Einsatz kommt. Durch letztere Kombination kann besonders vorteilhaft eine zusätzliche Einstellung der Menge der zerkleinerten Partikel pro Zeiteinheit durch die Regelung der Drehzahl der Dosierscheibe 30 erzielt werden.

Sowohl in der ersten als auch in der zweiten Ausführungsform der vorliegenden Erfindung kann die Einstellung der Menge der zerkleinerten Partikel pro Zeiteinheit am Mahlwerk und/oder der Transportwalze und/oder der Dosiereinrichtung durch einen oder mehrere (nicht gezeigte) Regler an der Strahlpistole durchgeführt werden.

Erfindungsgemäß wird auch ein Verfahren zur Oberflächenbearbeitung bzw. Oberflächenbehandlung mittels Trockeneisgranulat zur Verfügung gestellt, das die Zerkleinerung der herkömmlichen 3 mm Pellets auf die vorstehend genannten Größen vor der Bestrahlung der zu behandelnden bzw. zu verarbeitenden Fläche vorsieht.

Darüber hinaus sieht das erfindungsgemäße Verfahren Schritte vor, um die Menge der zerkleinerten Partikel pro Zeiteinheit am Mahlwerk und/oder der Transportwalze und/oder der Dosiereinrichtung einzustellen. Dieses kann, wie vorstehend erwähnt, durch einen oder mehrere (nicht gezeigte) Regler an der Strahlpistole erfolgen.

Ein weiteres Merkmal des erfindungsgemäßen Verfahrens ist die Bereitstellung von Schritten zur Einstellung der Form bzw. der Scharfkantigkeit der zerkleinerten Partikel, die wie vorstehend erwähnt an der Brechwalze des Mahlwerks und/oder an der Transportwalze erfolgen können.

Das erfindungsgemäße Verfahren erfüllt ebenfalls die gestellten Aufgaben und weist dieselben Vorteile, die in Verbindung mit der erfindungsgemäßen Vorrichtung erläutert wurden, auf.

Zusätzlich wurde überraschend vom gegenwärtigen Erfinder in Erfahrung gebracht, dass sich das Verfahren unter Anwendung der zerkleinerten Partikel sehr wirksam bei der kosmetischen Behandlung bzw. Reinigung von Haut eignet.

Ausdrücklich ausgeschlossen ist die Verwendung des Verfahrens zur Dermabrasion.

## Patentansprüche

1. Vorrichtung zur Oberflächenbearbeitung bzw. Oberflächenbehandlung mittels Trockeneisgranulat mit einem Vorratsbehälter (1) zur Aufnahme von Trockeneis-Pellets, einem Einlass (6) für die Zuführung von Druckluft an einen Strömungskanal (7), einem Auslass (22) zur Zuleitung eines Gemisches aus Druckluft und Trockeneisgranulat an eine Strahlpistole (9) und einer mit dazwischenliegenden Ausströmkammer (59), die mit dem Strömungskanal (7) verbunden ist,
wobei im Pfad zwischen dem Vorratsbehälter (1) und der Ausströmkammer (59) unterhalb des Vorratsbehälters (1) ein radiales Mahlwerk (3) angeordnet ist, das ausgebildet ist, um die durch die Schwerkraft aufliegenden Trockeneis-Pellets zu einer Größe in einem vorgegebenen Bereich zu zerkleinern,
das Mahlwerk (3) eine Brechwalze umfasst, die ausgebildet ist, um die Form der zerkleinerten Trockeneis-Pellets scharfkantig zu gestalten;
das Mahlwerk (3) ausgebildet ist die Größe der zerkleinerten Trockeneis-Pellets in einem Bereich von etwa 1 mm bis etwa 100 µ variabel einzustellen;
weiterhin eine radiale Transportwalze (4) umfasst, die zwischen dem Mahlwerk (3) und der Ausströmkammer (59) unterhalb de Mahlwerks (3) angeordnet ist, wobei die Transportwalze (4) mit einer veränderlichen Drehzahl betreibbar ist, um die Menge der unter Einwirkung der Schwerkraft vom Mahlwerk (3) auftreffenden zerkleinerten Trockeneis-Pellets pro Zeiteinheit zu variieren und um die zerkleinerten Trockeneis-Pellets in den Strömungskanal (7) zu befördern.

2. Vorrichtung nach Anspruch 1, **dadurch gekennzeichnet, dass** das Mahlwerk (3) ausgebildet ist die Größe der zerkleinerten Trockeneis-Pellets in einem Bereich von etwa 200 µ bis etwa 400 µ variabel einzustellen.

3. Vorrichtung nach Anspruch 1 oder 2, wobei die Brechwalze mit einer veränderlichen Drehzahl betreibbar ist, um die Menge der zerkleinerten
Trockeneis-Pellets pro Zeiteinheit zu variieren.

4. Vorrichtung nach einem der vorangehenden Ansprüche, **dadurch gekennzeichnet, dass** die Transportwalze (4) ausgebildet ist, um die Form der zerkleinerten Trockeneis-Pellets scharfkantig zu gestalten.

5. Verfahren zur Oberflächenbearbeitung bzw. Oberflächenbehandlung mittels Trockeneisgranulat mit einer Vorrichtung gemäß einem der vorangehenden Ansprüche.

6. Verwendung des Verfahrens nach Anspruch 5 für die kosmetische Behandlung bzw. Reinigung von Haut unter Ausschluss von Dermabrasion.

## Claims

1. A device for surface working or surface treatment, respectively, by means of dry ice granulate, having a reservoir (1) for receiving dry ice pellets, an inlet (6) for feeding compressed air to a flow passage (7), an outlet (22) for feeding a mixture of compressed air and dry ice granulate to a blast gun (9) and a discharge chamber (59) therebetween which is connected to the flow passage (7),
a radial mill work (3) being arranged in the path between the reservoir (1) and the discharge chamber (59) below the reservoir (1), which mill work is adapted to crush the dry ice pellets making contact by gravity to a size within a predefined range,
the mill work (3) comprising a crushing roll which is adapted to provide the crushed dry ice pellets with a sharp-edged shape;
the mill work (3) being adapted to variably adjust the size of the crushed dry ice pellets within a range of approximately 1 mm to approximately 100 µ;
further comprising a radial feed roll (4) arranged between the mill work (3) and the discharge chamber (59) below the mill work (3), where the feed roll (4) can be operated with variable rotational speed so as to vary the amount of crushed dry ice pellets per unit of time which make contact from the mill work (3) under the effect of gravity and so as to convey the crushed dry ice pellets into the flow passage (7).

2. The device according to Claim 1, **characterized in that** the mill work (3) is adapted to adjust the size of the crushed dry ice pellets variably within a range of approximately 200 µ to approximately 400 µ.

3. The device according to Claim 1 or 2, where the crushing roll can be operated with variable rotational speed so as to vary the amount of crushed dry ice pellets per unit of time.

4. The device according to one of the preceding claims, **characterized in that** the feed roll (4) is adapted to provide the crushed dry ice pellets with a sharp-edged shape.

5. A method for surface working or surface treatment, respectively, with dry ice granulate by means of a device according to one of the preceding claims.

6. A use of the method according to Claim 5 for the cosmetic treatment or cleaning, respectively, of skin, under the exclusion of dermabrasion.

## Revendications

1. Dispositif destiné au traitement de surface ou à la manipulation de la surface au moyen d'un granulé de glace sèche, avec un réservoir de stockage (1) pour recevoir des boulettes de glace sèche, une admission (6) pour l'amenée d'air comprimé à un canal d'écoulement (7), un échappement (22) pour l'amenée d'un mélange, composé d'air comprimé et de granulé de glace sèche, à un pistolet de projection (9), et une chambre de sortie d'écoulement (59), installée en position intermédiaire, reliée au canal d'écoulement (7),
dans le chemin situé entre le réservoir de stockage (1) et la chambre de sortie d'écoulement (59), au-dessous du réservoir de stockage (1), étant disposé un moulin (3) radial, réalisé pour broyer les boulettes de glace sèche, reposant sur lui sous l'effet de la force de la gravité, à une taille située dans une fourchette prédéterminée,
le moulin (3) comprenant un cylindre broyeur, réalisé pour donner aux boulettes de glace sèche broyées une forme à bords tranchants ;
le moulin (3) étant réalisé pour régler de manière variable la grosseur des boulettes de glace sèche broyées dans une fourchette d'à peu près 1 mm à à peu près 100 µm ;
en comprenant en outre un rouleau de transport (4) radial, disposé entre le moulin (3) et la chambre de sortie d'écoulement (59), au-dessous du moulin (3), le rouleau de transport (4) étant susceptible de fonctionner à une vitesse de rotation variable, pour faire varier la quantité, par unité de temps, de boulettes de glace sèche broyées, arrivant du moulin (3) sous l'effet de la force de la gravité et pour transporter les boulettes de glace sèche broyées dans le canal d'écoulement (7).

2. Dispositif selon la revendication 1, **caractérisé en ce que** le moulin (3) est réalisé pour régler de manière variable la grosseur des boulettes de glace sèche broyées dans une fourchette d'à peu près 200 µm à à peu près 400 µm.

3. Dispositif selon la revendication 1 ou 2, **caractérisé en ce que** le cylindre broyeur est susceptible de fonctionner à une vitesse de rotation modifiable, pour faire varier la quantité de boulettes de glace sèche broyées par unité de temps.

4. Dispositif selon l'une des revendications précédentes, **caractérisé en ce que** le rouleau de transport (4) est réalisé pour donner aux boulettes de glace sèche broyées une forme à bords tranchants.

5. Procédé destiné au traitement de surface ou à la manipulation de la surface au moyen d'un granulé de glace sèche, avec un dispositif selon l'un des revendications précédentes.

6. Utilisation du procédé selon la revendication 5, pour le traitement cosmétique ou le nettoyage de la peau, à l'exclusion de la dermabrasion.
